# EUROPEAN PATENT APPLICATION

(11) **EP 1 342 728 A1**
(43) Date of publication of application: **10.09.2003**
(21) Application number: 01983810.1
(22) Date of filing: 16.11.2001
(51) Int. Cl.: C07K 14/47, C12N 15/09, C12Q 1/68, A61K 31/711, A61K 38/00, A61K 48/00

(54) **RADIOSENSITIVITY ENHANCERS IN RADIOTHERAPY FOR CANCER AND NUCLEIC ACIDS AND PROTEINS ENHANCING RADIOSENSITIVITY**

(30) Priority: 17.11.2000 JP 2000351630
(71) Applicant: Chiba-Prefecture, Chiba-shi, Chiba 260-8667 (JP); HISAMITSU PHARMACEUTICAL CO. INC., Tosu-shi, Saga-ken 841-0017 (JP)
(72) Inventor: NAKAGAWARA, Akira, Chiba Cancer Center Res. Insti., Chiba-shi, Chiba260-0 801 (JP)
(74) Representative: Grünecker, Kinkeldey, Stockmair & Schwanhäusser Anwaltssozietät
(86) International application number: JP0110036
(87) International publication number: WO02040533

(57) **Abstract**

A radiosensitivity enhancer comprising as the effective ingredient, a nucleic acid having the base sequence set forth in SEQ ID NO:1 or SEQ ID NO:3, or a protein having the amino acid sequence set forth in SEQ ID NO:2 or SEQ ID NO: 4 is used to enhance the radiosensitivity of a malignant tumor cell with the sensitization effect allowing radiotherapy to be effective against a radiation resistant tumor or the malignant tumor of which the radiosensitivity has been lowered.

## Description

### Technical Field

This invention relates to radiosensitivity enhancers, as well as to radiosensitivity-enhancing nucleic acids and proteins in cancer radiotherapy. More particularly, it relates to nucleic acid information of genes in the homozygous deletion region that may be commonly present at position 36 on the short arm of chromosome 1 (hereafter referred to as "1p36") of human neuroblastoma, as well as to the therapeutic or diagnostic utility of the nucleic acids and proteins encoded by said nucleic acids (especially, enhancement of the radiosensitivity of cancer cells in the cancer radiotherapy).

### Background Art

Conventionally, therapy against cancer (malignant tumor) has been conducted, including surgical treatment, chemotherapy, immunotherapy, hiperthermia treatment, and radiotherapy. The radiotherapy can be employed alone or in combination with other treatment methods, and the therapy is an effective treatment method since it shows a high local controlling rate in many malignant tumors.

The radiosensitivity of tumor cells is known to decrease with lowering oxygen concentration. In order for the radiotherapy to be effective, it is assumed that with respect to radiosensitivity, tumor cells are greater than normal cells in the region to be treated. Thus, the development of pharmaceutical agents for enhancing the radiosensitivity of the tumor cells in place of oxygen is progressing. One example of such pharmaceutical agents is referred to as "a hypoxic cell radiation sensitizer"; and it displays no enhancement of the radiosensitivity of peroxic cells (normal cells), but has enhancing effect only on hypoxic cells (tumor cells). Nitroimidazole derivatives are recognized to be the radiation sensitizers, and compounds such as misonidazole and ethanidazole have been developed. However, they have not resulted in the practical use because their nerve toxicity is too strong at the doses providing sensitization activity. Although the combined use of the pharmaceutical agent for enhancing radiosensitivity is desired in the treatment of a radiation resistant tumor, many of the radiosensitivity enhancers (such as radiation sensitizers) experience a problem in their development because of nerve toxicity.

It is recognized that inhibitors of nucleic acid or protein synthesis such as actinomycin D, methotrexete, and hyromycin and halogen derivatives of nucleic acid base such as 5-fluorouracil are effective in enhancing radiosensitivity regardless of the presence of oxygen. These, however, exert their effectiveness by suppressing the normal function of the cells.

Furthermore, it has been reported that TNF (tumor necrosis factor) can enhance the radiosensitivity of a certain tumor cell under particular conditions. Hallahan et al. showed that addition of TNF to SCC-61 tumor cells 4-8 hours prior to irradiation could enhance killing power on the tumor cells *in vitro*. See, Hallahan et al., Important Advances in Oncology, pp. 75-76 (1993). There is also a report that pretreatment with TNF can enhance the radiosensitivity of the human leukemia cell lines, HL-60, K562 and U937. Wong et al., Eicosanoids and Other Bioactive Lipids in Cancer, Inflammation and Radiation Injury, S. Nigam, ed. (1993), Ch. 70 pp. 353-357. However, the mechanism remains unknown, including whether or not TNF after all enhances the radiosensitivity of a specific tumor cell.

In contrast, a pharmaceutical agent, "radiation-protecting agent" is also contemplated, which possesses the action opposite to "the radiosensitivity enhancer." This is a pharmaceutical agent that aims at neutralizing free radicals generated by radiation and at accelerating the recovery of tissue from radiation injury. Specifically, a group of compounds having a SH group such as cysteine are being screened. The radiation-protecting agent lowers the radiosensitivity and substantially increases tolerable doses. These pharmaceutical agents, therefore, have the possibility that they will be useful for the treatment of radiation injuries such as internal radiation exposure or myelopathy.

Human neuroblastoma (also referred to as simply "neuroblastoma" hereafter) is a malignant tumor originating from precursor cells of the fetal sympathetic nervous system, and is most frequently seen among the pediatric solid cancers. The treatment for this tumor is basically surgical excision, but in advanced cases chemotherapy or radiotherapy may be selected. Although neuroblastoma is a tumor that has high radiosensitivity, reduction in the radiosensitivity will be observed as the treatment progresses. However, with respect to neuroblastoma the mechanism of radiosensitivity has hardly been elucidated, and no solution has been found where the radiotherapy meets a difficulty.

### Disclosure of the Invention

This invention has been accomplished in light of the circumstances described above. An object of the invention is to elucidate the mechanism on how the radiosensitivity of a malignant tumor is lowered in radiotherapy and to prevent the radiosensitivity from being lowered based on the elucidation. Further, another object is to reveal the information on genes involved in the mechanism, to enhance the radiosensitivity of malignant tumor cells based on the information, and to provide a radiosensitivity enhancer for improving the therapeutic rate of the radiotherapy.

In accordance with the above-stated objects, the present inventors repeated studies diligently, and consequently, discovered that the radiosensitivity of certain human neuroblastoma strains is extremely low as compared to other strains. Further, they identified a group of genes lacking in the former strains and confirmed that the deletion of these genes lowered the radiosensitivity and oppositely the insertion of the genes enhanced the radiosensitivity. In this connection, the group of genes was found to at least contain PEX14 gene and a gene encoding a novel UFD2 associated protein (referred to as "HDNB-1/UFD2" hereafter). The cDNA sequence (base sequence) of HDNB-1/UFD2 and the amino acid sequence encoded by the gene are, respectively, set forth in SEQ ID NO:1 and SEQ NO:2. The cDNA sequence (base sequence) of PEX14 and the amino acid sequence encoded by the gene are, respectively, set forth in SEQ ID NO:3 and SEQ NO:4.

From such findings, the present inventors made it possible to control the radiosensitivity of the malignant cells, and completed this invention.

In summary, this invention provides the therapeutic or diagnostic use of the nucleic acids and proteins (including partial peptides thereof) or their pharmaceutically acceptable salts described in 1-16 below.
1. A nucleic acid having the base sequence set forth in SEQ ID NO:1 in the Sequence Listing.
2. A nucleic acid encoding a protein comprising the amino acid sequence set forth in SEQ ID NO:2 in the Sequence Listing.
3. A protein having the amino acid sequence set forth in SEQ ID NO:2 in the Sequence Listing.
4. A radiosensitivity enhancer comprising as the effective ingredient, a nucleic acid having the base sequence set forth in SEQ ID NO:1 in the Sequence Listing.
5. A radiosensitivity enhancer comprising as the effective ingredient, a nucleic acid encoding a protein comprising the amino acid sequence set forth in SEQ ID NO:2 in the Sequence Listing.
6. A radiosensitivity enhancer comprising as the effective ingredient, a nucleic acid having the base sequence set forth in SEQ ID NO:3 in the Sequence Listing.
7. A radiosensitivity enhancer comprising as the effective ingredient, a protein having the amino acid sequence set forth in SEQ ID NO:2 in the Sequence Listing or a pharmaceutically acceptable salt thereof.
8. A radiosensitivity enhancer comprising as the effective ingredient, a partial peptide which is a functionally effective fragment of the protein having the amino acid sequence set forth in SEQ ID NO:2 in the Sequence Listing or a pharmaceutically acceptable salt thereof.
9. A radiosensitivity enhancer comprising as the effective ingredient, a protein having the amino acid sequence set forth in SEQ ID NO:4 in the Sequence Listing or a pharmaceutically acceptable salt thereof.
10. A radiosensitivity enhancer comprising as the effective ingredient, a partial peptide which is a functionally effective fragment of the protein having the amino acid sequence set forth in SEQ ID NO:4 in the Sequence Listing or a pharmaceutically acceptable salt thereof.
11. A recombinant viral vector comprising a nucleic acid having the base sequence set forth in SEQ ID NO:1 or SEQ ID NO:3 in the Sequence Listing alone, or in combination with an appropriate regulatory and expression control element operably linked thereto.
12. A nucleic acid probe comprising the following nucleic acid (a) or (b):
   (a) Nucleic acid having a portion of the base sequence set forth in SEQ ID NO:1 in the Sequence Listing, or a base sequence complementary thereto;
   (b) Nucleic acid which hybridizes to nucleic acid having the base sequence set forth in SEQ ID NO:1 in the Sequence Listing under stringent conditions, or a base sequence complementary thereto.
13. A nucleic acid probe comprising the following nucleic acid (a) or (b):
   (a) Nucleic acid having a portion of the base sequence set forth in SEQ ID NO:3 in the Sequence Listing, or a base sequence complementary thereto;
   (b) Nucleic acid which hybridizes to nucleic acid having the base sequence set forth in SEQ ID NO:3 in the Sequence Listing under stringent conditions, or a base sequence complementary thereto.
14. A method for determining the sensitivity of a clinically isolated human neuroblastoma strain to radiotherapy, the method comprising detecting a nucleic acid having the base sequence set forth in SEQ ID NO:1 or SEQ ID NO:3 or a fragment thereof in the human neuroblastoma strain.
15. A radiation-protecting agent comprising as the effective ingredient, an antisense nucleic acid to the nucleic acid having the base sequence set forth in SEQ ID NO:1 or SEQ ID NO:3 in the Sequence Listing.
16. A method for enhancing the radiosensitivity of human neuroblastoma in the radiotherapy of a patient in need of such treatment for the human neuroblastoma, the method comprising administering to the patient, an effective amount of a nucleic acid having the base sequence set forth in SEQ ID NO:1 or a protein having the amino acid sequence set forth in SEQ ID NO:2, or a pharmaceutically acceptable salt thereof.
17. A method for enhancing the radiosensitivity of human neuroblastoma in the radiotherapy of a patient in need of such treatment for the human neuroblastoma, the method comprising administering to the patient, an effective amount of a nucleic acid having the base sequence set forth in SEQ ID NO:3 or a protein having the amino acid sequence set forth in SEQ ID NO:4, or a pharmaceutically acceptable salt thereof.
18. A method for enhancing the radiosensitivity of human neuroblastoma in the radiotherapy of a patient in need of such treatment for the human neuroblastoma, the method comprising coadministering to the patient, an effective amount of a nucleic acid having the base sequence set forth in SEQ ID NO:1 and a nucleic acid having the base sequence set forth in SEQ ID NO:3, or alternatively a protein having the amino acid sequence set forth in SEQ ID NO:2, or a pharmaceutically acceptable salt thereof and a protein having the amino acid sequence set forth in SEQ ID NO:2, or a pharmaceutically acceptable salt thereof.

### Brief Description of the Drawings

Fig. 1 is an alignment diagram comparing the putative amino acid sequence for HDNB-1/UFD2 which is a gene according to this invention and the putative amino acid sequence for human UFD2 (AF043117) which has been registered in a database.
Fig. 2 is a representation of electropherogram showing the results of expression of HDNB-1/UFD2 gene in normal human tissue as detected by semi-quantitative RT-PCR.
Fig. 3 is a representation of electropherogram showing the ubiquitin activity of HDNB-1/UFD2.
Fig. 4 is a plot of the cell survival rate of each strain against the number of elapsed days post-irradiation when neuroblastoma strain C201 or other neuroblastoma strains having PEX14 and HDNB-1/UFD2 genes introduced were irradiated.
Fig. 5 is a bar graph comparing the respective radiosensitivities when plural C201(HDNB-1/UFD2) strains and the control C201(neo) strain have been irradiated and the time-dependent changes in the cell survival rates of the respective strains are plotted.

### Best Mode for Carrying Out the Invention

The phrase, "protein having the amino acid sequence set forth in SEQ ID NO:2" as used in the present specification may refer not only to a protein encoded by the nucleic acid set forth in SEQ ID NO:1, but also to any protein having substantially the same amino acid sequence as that set forth in SEQ ID NO:2 with substantially equivalent activity (which enhances radiosensitivity) to said protein. The substantially same amino acid sequence is, for example, an amino acid sequence derivable by the substitution or the deletion of one or more amino acids in the amino acid sequence set forth in SEQ ID NO:2, or by the addition of one or more amino acids to the amino acid sequence set forth in SEQ ID NO:2. Also, the phrase "protein having the amino acid sequence set forth in SEQ ID NO:4" has an exactly corresponding meaning, and may refer not only to a protein encoded by the nucleic acid having the base sequence set forth in SEQ ID NO:3, but also to any protein with substantially equivalent activity (which enhances radiosensitivity).

The phrase, "nucleic acid having the base sequence set forth in SEQ ID NO:1" as used in the present specification may also refer to nucleic acid having substantially the same base sequence as that set forth in SEQ ID NO:1 with substantially equivalent activity (which enhances radiosensitivity) to said nucleic acid. The substantially same base sequence is, for example, nucleic acid encoding the above-mentioned variant protein. The phrase "nucleic acid having the base sequence set forth in SEQ ID NO:3" has an exactly corresponding meaning, and may also refer to nucleic acid having substantially the same base sequence as that set forth in SEQ ID NO:3 with substantially equivalent activity to said nucleic acid. Such nucleic acids and protein variants may be prepared according to techniques known to one skilled in the art such as site-specific mutation, based on the base sequence information of the above-mentioned nucleic acids.

The term, "base sequence encoding a protein" as used in the present specification is an expression in consideration of degeneracy and it may refer to a base sequence different from the base sequence represented by cDNA corresponding to said protein. In other words, plural base sequences (codons) exist that encode one amino acid; therefore, the base sequence encoding a protein comprising the amino acid sequence set forth in SEQ ID NO:2 is not singular and it is not limited to the base sequence set forth in SEQ ID NO:1. Thus, the term encompasses base sequences encoding a protein comprising the amino acid sequence set forth in SEQ ID NO:2 other than the base sequence set forth in SEQ ID NO:1.

The term "nucleic acid" as used in the present specification refers to DNA or RNA which encodes a protein as defined above or a partial peptide as a functionally effective fragment of the protein, which is complementary to a nucleic acid encoding such a protein or partial peptide, or which hybridizes to such nucleic acid under "stringent" conditions. The forms of such DNAs include genomic DNA, cDNA and mRNA. When the "nucleic acid" means DNA, it is typically represented by a single-stranded DNA (sense strand) as shown by the sequence identification numbers described above, but the definition of "nucleic acid" also includes a single-strand complementary to the sequence (antisense strand) and double-stranded DNA comprising both strands.

The "hybridize under stringent" conditions as described above are described by Sambrook, J. et al. in "Expression of cloned genes in *E*. *coli*", Molecular Cloning: A Laboratory Manual (1989), Cold Spring Harbor Laboratory Press, New York, USA, 9.47-9.62 and 11.45-11.61. More specifically, they refer to hybridization at approximately 45°C, 6.0 x SSC, followed by washing at 50°C, 2.0 x SSC. The stringency may be selected by choosing a salt concentration in the washing step from approximately 2.0 x SSC, 50°C as low stringency to approximately 0.2 x SSC, 50°C as high stringency. Also, the temperature in the washing step may be increased from room temperature, or approximately 22°C as low stringency conditions, to approximately 65°C as high stringency conditions.

As used in the present specification, "radiosensitivity" means the degree of tumor cells being killed by radiation. The "radiosensitivity enhancer" refers to a pharmaceutical agent capable of enhancing (or improving) radiosensitivity (which may also be referred to as "radiosensitiser") regardless of the mechanism of its action: it is not restricted to a radiosensitiser against hypoxic cells. The "radiation-protecting agent" refers to a pharmaceutical agent capable of lowering radiosensitivity regardless of the mechanism of its action.

The construction and preferred embodiments of this invention will be described in detail hereinafter.

In neuroblastoma the deletion of 1p36 occurs in high frequency, and it has been predicted that cancer-controlling genes may be present in this 1p36 region. The present inventors analyzed approximately 400 cases of neuroblastoma for LOH (Loss of Heterozygosity) in lp36. Consequently, a common deletion region (about 8 to 10 Mb) was found. In addition, when genomic DNA was extracted from numerous neuroblastoma-derived cell lines and screened, Loss of Homozygosity was discovered in the common deletion region with respect to two cell lines, NB1 and MASS-NB-SCH-1. Thus, it was thought that critical genes for controlling the tumorgenesis and biological characteristics of neuroblastoma are present in this common region.

PAC clones were used to construct contings for the purpose of acquiring genetic information which includes sequencing for the common deletion region, the detail of which is disclosed in International Patent Publication PCT/JP00/05930. Further, several primer sets used for the construction of the contings were employed as keywords to search the gene mapping database (http://www.ncbi.nlm.nih.gov/genemap99/) and BLAST (http://www.ncbi.nlm.nih.gov/BLAST), when a number of known genes were hit and these were confirmed to be present in the common deletion region. The base sequence information for the genes is summarized in Table 1.

**Table 1**

| Base Sequence Information for the Deletion Region | | | |
|---|---|---|---|
| GENE | NAME OR ACCESSION No. | DEFINITION AND CHARACTERISTICS | ORF(bp) |
| 1 | UFD2(AF043117) | ubiquitin-fusion degradation protein 2 | 3909 |
| 2 | KIF1Bβ | kinesin-like protein KIF1B | |
| 3 | CORT(NM001302) | preprocortistatin | 318 |
| 4 | PEX-14(NM004565) | peroxisomal biogenesis factor 14mRNA | 1908 |
| 5 | KIAA0684(AB014584) | Homo sapiens clone 686 protein(KIAA0684) mRNA | 3281 |
| 6 | KIAA0591(AB011163) | Homo sapiens mRNA for KIAA0591 protein, partial cds. | 5368 |
| 7 | AI080353(EST) | ox80f04.s1 Soares_NhHMPu_S1 Homo sapiens CDNA clone | 422 |
| 8 | D79099(EST) | HUM531D06B Human placenta polyA+(TFujiwara) Homo sapiens cDNA clone | 320 |
| 9 | AA059029(EST) | Zf63h02.s1 Soares retina N2b4HR Homo sapiens cDNA clone | 446 |
| 10 | AA628589(EST) | af39g05.s1 Soares_total_fetus_Nb2HF8_9w Homo sapiens CDNA clone | 443 |
| 11 | AA728950(EST) | nw02c08.s1 NCI_CGAP_Pr16 Homo sapiens cDNA clone | 437 |
| 12 | AA328945(EST) | EST32514 Embryo, 12 week\|Homo sapiens cDNA 5'end, mRNA sequence | 302 |
| 13 | R89689(EST) | ym99c01.r1 Soares adult brain N2b4HB55Y Homo sapiens cDNA clone | 450 |
| 14 | AA018367(EST) | Ze41e05.r1 Soares retina N2b4HR Homo sapiens cDNA clone | 504 |
| 15 | Z19845(EST) | HSAAAAUKT H, Human adult Brain Cortex tissue Homo sapiens CDNA | 281 |
| 16 | AI050021(EST) | an22d04.x1 Gessler Wilms tumor Homo sapiens cDNA clone | 434 |
| 17 | R67760(EST) | yi28f03.r1 Soares placenta Nb2HP Homo sapiens cDNA clone | 496 |

The present inventors took note of PEX14 and UFD2 in this gene group of genes and investigated their functions in the neuroblastoma cells. During the process of investigation, a finding was obtained that the genes encoding these two types of proteins having completely different physiological activities are both deeply involved in the radiosensitivity of the cells. This invention is then based on the finding.

Human PEX14 (HsPEX14) is a gene encoding human peroxisome binding protein (HsPeX14p) and the protein is known to be the central protein involved in the transport mechanism for peroxisome proteins. Further, human PEX14 is believed to be a candidate gene for diseases associated with peroxisome-biogenesis. The complete cDNA sequence of human PEX14 and the putative amino acid sequence of HsPex14p have both been reported in G. K. Will et al., Molecular and Cellular Biology 19, 2265-2277 (1999). In addition, the identical sequences have been registered with GenBank at NCBI (http://www.ncbi.nlm.nih.gov.) as Accession No. NM004565.

UFD2 is a protein that is isolated from yeast and that participates in the ubiquitin pathway, but its function has not fully been elucidated. Recently, it has been shown that UFD2 is identical to the ubiquitinzation factor E4 which cooperates with the ubiquitin-activating enzyme E1, the ubiquitin-binding enzyme E2 and the ubiquitin-ligating enzyme E3 in the ubiquitin-ligating system (Koegl et al., Cell, 96, 635-644 (1999)). UDF2 (E4) is responsible for elongating the polyubiquitin chain when the target protein is subjected to ubiquitinzation by the aforementioned enzyme group. The polyubiquinated protein is then decomposed by proteasome. Thus, the ubiquitin/proteasome system plays a central role in the cellular proteolysis. KIAA0684 protein that had been registered by Nagase et al. (GenBank (http://www.ncbi.nlm.nih.gov) Accession No. AB014584) was identified as a human homolog to yeast UDF2 (Koegl et al., loc. cit.). The full amino acid sequence of human UDF2 which is longer in chain and cDNA sequence encoding it has been registered with GenBank (http://www.ncbi.nlm.nih.gov) as Accession No. AF043117 (by B. Lubyova et al.).

However, the gene of which the expression was confirmed in several neuroblastoma strains by the present inventors lacked in a portion of the sequence registered as AF043117, and thus, was shorter than said sequence by 387 bp. In addition, the gene sequence showed complete identity with the aforementioned registered sequence except for this deleted region. Therefore, this gene was designated HDNB-1/UFD2. Of note was that the expression of UDF2 by Lubyova et al. had not been observed in the neuroblastoma cell lines tested (CHP134, GOTO/P3, SKN-SH, and TGW) at all but only the expression of HDNB-1/UFD2 had been observed.

The gist of this invention is to use HDNB-1/UFD2 (SEQ ID NO:2), PEX14 (SEQ ID NO:4) (where both proteins do not need to be distinguished from each other, they will be individually or collectively referred to as "protein(s) of this invention" hereafter), or nucleic acids of the genes encoding the respective proteins (where both nucleic acids do not need to be distinguished from each other, they will be individually or collectively referred to as "nucleic acid(s) of this invention" hereafter) as radiosensitivity enhancers in cancer radiotherapy.

Further, it was discovered that when either or both of the genes according to this invention were lacking in the lp36 homozygosity deletion region of neuroblastoma cell lines, the radiosensitivity of such neuroblastoma cell lines was low.

Accordingly, the proteins according to this invention (including partial peptides which are their effective fragments), the nucleic acids according to this invention (including their fragments) and antisense nucleic acids to said nucleic acids can be used for the diagnosis of neuroblastoma and other cancers as well as in radiotherapy.

### (1) Usefulness for diagnosis

In accordance with this invention, the proteins, nucleic acids and antibodies for the proteins are provided with usefulness for diagnosis as described below.

Specifically, these molecules may be used for determination of the radiosensitivity of a neuroblastoma strain by measuring the level of their expression in the neuroblastoma strain through various assays.

There are no particular limitations on the methods of immunoassay using antibodies for the proteins according the invention, and there may be mentioned various competitive and non-competitive assays using such techniques as Western blotting, radioimmunoassay, ELISA, "sandwich" immunoassay, immunoprecipitation, precipitin reaction, gel differentiation precipitation reaction, immunodiffusion assay, agglutination assay, complement-binding assay, immunoradiometric assay, fluorescent immunoassay and protein A immunoassay.

When a nucleic acid according to the invention is to be used in diagnosis, it may be used as a hybridization probe or as a PCR primer to examine the expression level of the gene (i.e., the gene according to the invention) in a neuroblastoma cell sample, which will enable the radiosensitivity of said cell to be determined. The presence of enhancement in gene expression can be examined, for example, by any conceivable method using as the probe a base sequence which hybridizes to any desired sequence of the nucleic acid according to the invention. Preferably, a radioactive isotope-labeled probe is used for assay by Southern or Northern blotting. When the nucleic acid is used as a primer for PCR, RNA may be, for example, extracted from the sample (cells) to be assayed and the gene expression may be semi-quantitatively measured by RT-PCR.

### (2) Usefulness for treatment

The nucleic acids and proteins according to the invention are radiosensitivity enhancers; therefore, they are provided with usefulness as adjuvants in cancer radiotherapy (particularly, against neuroblastoma).

Typically, a pharmaceutical composition comprising a nucleic acid or a protein according to the invention may be administered to such a patient in need of treatment in an effective amount (i.e., appropriate dose) prior to radiotherapy. The nucleic acid having the base sequence set forth in SEQ ID NO:1 and the nucleic acid having the base sequence set forth in SEQ ID NO:3 may individually be included in the pharmaceutically composition and may be administered separately. Alternatively, they may be both included in the pharmaceutical composition and may be administered as a combined agent of the two. In a similar manner, the protein having the amino acid sequence set forth in SEQ ID NO:2 or a pharmaceutically acceptable salt thereof and the protein having the amino acid sequence set forth in SEQ ID NO:4 or a pharmaceutically acceptable salt thereof may individually be included in the pharmaceutically composition and may be administered separately; or alternatively, they may be both included in the pharmaceutical composition and may be administered as a combined agent of the two.

Where the expression level of the protein according to the invention is increased, a pharmaceutical composition comprising an antisense nucleic acid, neutralizing antibodies or a competitive inhibitor for said protein may be administered; thus, it will be possible to either suppress the expression level or inhibit the function of the protein. As a result, the patient administered with such a pharmaceutical composition will experience lowered radiosensitivity.

Particularly when a nucleic acid of the invention is used as a radiosensitivity enhancer for the purpose described above, the nucleic acid may be inserted into a vector used for gene transfer and the gene is allowed for expression as a transgene in the body of the patient under any desired expression promoter, followed by radiotherapy. The nucleic acid having the base sequence set forth in SEQ ID NO:1 and the nucleic acid having the base sequence set forth in SEQ ID NO:3 may individually be inserted into the vector, or alternatively they may be inserted concurrently.

The vector for insertion of the nucleic acid is preferably constructed based on a DNA or RNA virus. There are no particular limitations on the type of virus vectors, and there may be used an MoMLV vector, herpes virus vector, adenovirus vector, AAV vector, HIV vector, SIV vector, Sendai virus vector and the like.

There may be used, alternatively, a pseudotyped virus vector wherein one or more of the constitutive proteins of the virus vector is replaced with a constitutive protein of a different type of virus, or wherein a portion of the nucleic acid sequence of the genetic information is replaced with a nucleic acid sequence of another type of virus. As an example, there may be mentioned a pseudotyped virus vector wherein Env protein, the coat protein of HIV, is replaced with VSV-G protein, the coat protein of Vesicular Stomatitis Virus (VSV) (Naldini L. et al., Science 272:263- (1996)).

So long as the virus is capable of gene transfer, it may be used as a virus vector even if its host range is other than human. Non-virus-derived vectors may also be used, including calcium phosphate/nucleic acid complexes, liposomes, cationic lipid complexes, Sendai virus liposomes, and polymer carriers with polycationic backbone. Further, electroporation, a gene gun, or the like may be used as the gene transfer system.

An expression cassette provided with an expression promoter is preferably used to insert the nucleic acid of the invention inserted into the aforementioned vector for gene expression.

The expression cassette used may be of any type that allows gene expression in target cells, with no particular limitations. One skilled in the art can easily select such an expression cassette, which is preferably an expression cassette allowing gene expression in animal-derived cells, more preferably an expression cassette allowing gene expression in mammal-derived cells and even more preferably an expression cassette allowing gene expression in human-derived cells.

The expression cassette may include, in addition to the nucleic acid of the invention, various sequences such as a promoter or enhancer for the gene transcription, a polyA signal, a marker gene for labeling and/or selecting the gene-inserted cells, a viral gene sequence for efficient insertion of the gene into the genomic DNA sequence of the cell, and a signal sequence for extracellular secretion and/or local intracellular accumulation of the proteins produced by the gene expression.

For promoters to be used in the expression cassette, there may be mentioned promoters derived from such viruses as adenovirus, cytomegalovirus, human immunodeficiency virus, simian virus 40, Rous sarcoma virus, herpes simplex virus, mouse leukemia virus, sindbis virus, hepatitis A virus, hepatitis B virus, hepatitis C virus, papillomavirus, human T cell leukemia virus, influenza virus, Japanese encephalitis virus, JC virus, parvovirus B19 and poliovirus, mammalian promoters such as albumin, SRα, heat shock protein and elongation factor promoters, chimeric promoters such as CAG promoter, and promoters whose activity is induced by tetracycline, steroids and the like.

### (3) Pharmaceutical compositions

The nucleic acids and proteins of this invention are provided for radiotherapy in the form of appropriate pharmaceutical compositions. For this purpose, the nucleic acids or the like are prepared into formulation according to the formulation method described below, the preferred route of administration is established, and the dosage is determined so as to achieve the desired enhancing effect of radiosensitivity.

### (Formulation method)

The pharmaceutical composition comprising a nucleic acid or a protein according to the invention is not particularly limited, and a drug may be constructed by encapsulation in liposomes, fine particles or microcapsules, expression in recombinant cells, receptor-mediated ingestion, or as a retrovirus or a portion of another type of vector.

More specifically, a recombinant virus vector comprising the nucleic acid of the invention may be dissolved in an appropriate solvent such as water, physiological saline or an isotonized buffer solution to prepare a composition containing the nucleic acid of the invention. Alternatively, the protein of the invention may be dissolved in an appropriate solvent such as water, physiological saline or an isotonized buffer solution to prepare a composition containing the protein of the invention. Polyethylene glycol, glucose, various amino acids, collagen, albumin or the like may be then added as protective materials for the preparation.

The aforementioned pharmaceutical composition may contain pharmaceutically acceptable carriers, diluents, fillers, or stabilizers, if desired and this is often preferable. As to these excipients in formulation, they are fully described in Remington's Pharmaceutical Sciences, Mack Publisher, 16 (1980).

The protein of the invention may be formulated into a pharmaceutical composition as the form of a pharmaceutically acceptable salt. Pharmaceutically acceptable salts include those formed with the free amino group of a protein or peptide, such as those derived from hydrochloric acid, phosphoric acid, acetic acid, oxalic acid, tartaric acid or the like, and those formed with the free carboxyl group of a protein or peptide, such as those derived from sodium, potassium, ammonium, calcium, iron (II) hydroxide, isopropylamine, triethylamine, 2-ethylaminoethanol, histidine, procaine or the like.

### (Administration method and dosage)

The pharmaceutical composition according to this invention results in desired radiosensitivity-enhancing effect when an appropriate route of administration for the composition is set and it is administered to a patient. In administering the pharmaceutical composition to the body, there are no particular limitations on the method of administration. Nevertheless, it may be preferably carried out by injection intradermally, intramuscularly, intraperitoneally, intravenously, hypodermically or intranasally. Other administration methods such as infusion that allows continuous administration to the blood stream are also preferred. The particularly preferred mode of administration is intravenous administration. The dosage of the pharmaceutical composition of the invention will depend on the route of administration and the condition, age, body weight, gender, etc. of the patient receiving administration, and the optimum dosage for the patient may be determined by the practicing physician. In the case of injection, for example, the dosage is preferably about 0.1 µg/kg to 1000 mg/kg per day, and more preferably about 1 µg/kg to 100 mg/kg per day.

### (4) Radiotherapy

The radiotherapy contemplated by this invention is generally used in the field of art to which the invention pertains and may be carried out according to the protocol known to one skilled in the art. For example, the radiotherapy includes cesium-, iridium-, iodine- or cobalt-irradiation. The radiotherapy may be systemic irradiation (for acute leukemia, malignant lymphoma, certain solid cancers), but is preferably by way of local irradiation to the site or the tissue of tumor (the abdominal part, lung, liver, lymph nodes, or head against solid cancer). Typically, the radiotherapy is carried out for two to three minutes daily in 25 to 30 installments (approximately five to six weeks).

According to a preferred embodiment of the invention, the pharmaceutical composition of this invention is administered to the patient prior to the radiotherapy. When the radiotherapy is to be continuously conducted, the administration can be continuous as well.

The radiosensitivity enhancer of the invention may be used as an adjuvant in combination with the radiotherapy against a malignant tumor with inherently low radiosensitivity or a malignant tumor having acquired radiation resistance as a result of the radiotherapy.

The radiosensitivity enhancer of the invention can also lower the dose to be therapeutically applied by enhancing the radiosensitivity of a tumor cell and can extend the treatment period (exposure time) over the period prescribed by the ordinary protocol. Furthermore, it alleviates side effects due to radiation injuries (such as stomatitis, myelopathy, radiation ulcer, and radiation pneumonitis) necessarily accompanying the radiotherapy.

### (5) Targeted cancers

There are no particular limitations on the targeted cancers against which the radiosensitivity enhancer of this invention may be used as an adjuvant to radiotherapy, so long as the conventional radiotherapy can be conducted on the cancers. The radiosensitivity enhancer of this invention is particularly suited to the cancers the radiosensitivity of which is said to be high; but even in the cases of cancers the radiosensitivity of which is said to be low, the radiosensitivity enhancer can be expected to improve the therapeutic effect of radiotherapy since it can enhance radiosensitivity.

Malignant tumors as targets of treatment by the radiosensitivity enhancer of this invention include the following representatives: specifically, there may be mentioned acute leukemia, chronic leukemia, medulloblastoma, retinoblastoma, neuroblastoma, Wilms tumor, lymphoma, Hodgkins disease, multiple myeloma, plasmacytoma, thyroid carcinoma, basalioma, squamous cell carcinoma (cervical carcinoma), breast carcinoma, Ewing tumor, lung carcinoma, esophagus carcinoma, larynx carcinoma, thyroid carcinoma, ovarian carcinoma, sialadenoncus, teratoma, melanoma, glioma, stomach cancer, intestinal cancer, colon carcinoma, renal cell carcinoma, osteosarcoma, among others.

### (6) Antisense nucleic acids

According to another embodiment of this invention, antisense nucleic acid is used, which suppresses the expression of a gene according to the invention, thus achieving a therapeutic effect. Here, "antisense nucleic acid" refers to a nucleic acid that can hybridize to a portion of RNA (preferably mRNA) of a gene according to the invention due to a certain degree of sequence complementarity.

The antisense nucleic acid may be used in any form of RNA and DNA (encoding the RNA) oligonucleotides, whether a double-stranded or single-stranded, triple strand-forming oligonucleotide, or a chimeric mixture of the foregoing. The antisense nucleic acid is not particularly limited, and may consist of an oligonucleotide of preferably 5-200 bases. The oligonucleotide may also be modified in its base portion, ribose portion or phosphate backbone.

As a specific embodiment, the antisense nucleic acid may be used in the form of a catalytic RNA, ribozyme, or a chimeric RNA-DNA analog.

The antisense nucleic acids may be synthesized by a method known to one skilled in the art using, for example, an automated DNA synthesizer.

Specifically, such antisense DNA has a DNA sequence that is complementary to at least a part of the base sequence set forth in SEQ ID NO:1 or SEQ ID NO:3. Such antisense RNA has an RNA sequence that is complementary to at least a part of RNA encoded by DNA comprising the base sequence set forth in SEQ ID NO:1 or SEQ ID NO:3.

The antisense nucleic acid according to the invention hybridizes to the DNA or the RNA that carries genetic information during the process of production of the protein according to this invention from the gene according to the invention (transcription, translation and etc.), and affects the normal flow of transmission of the genetic information, thus inhibiting the biosynthesis of said protein. Therefore, the cell where the gene according to the invention is present experiences lowered radiosensitivity by the introduction of the antisense nucleic acid.

When the antisense nucleic acid is used as a radiation-protecting agent for the purpose of treatment, it may be administered to a patient as a pharmaceutical composition in the same manner described above for other nucleic acids, but most preferably, it is directly administered to specific cells. Cells may also be transformed with a vector comprising DNA encoding RNA antisense nucleic acid, or transfected, to produce the antisense nucleic acid in the cells by transcription.

### (7) Antibodies

According to yet another embodiment of this invention, antibodies against a protein according to the invention, or fragments thereof including the binding domains, may be used as a diagnostic agent as described above. Specifically, the antibodies may be used in various types of immunoassays for the detection and measurement of the protein according to the invention.

The antibodies may be prepared using the protein according to the invention, or its fragment, analog or derivative, as an immunogen by methods known to one skilled in the art. For such antibodies, there may be mentioned polyclonal antibodies, monoclonal antibodies, chimeric antibodies, single-stranded antibodies, Fab fragments, or antibodies derived from an FAB expression library.

### Examples

The invention will be described in greater detail by way of examples hereafter; however, these examples are in no way limitative on the invention.

### (Example 1) Cloning of HDNB-1/UFD2

Two types of primers, 5'-GTGTTGGAGGCCAAATGTG-3' and 5'-TTGACCGTGTTTGTCACTTAAA-3', were designed based on the sequence of SGC30343 which was a STS maker obtained from a database; and a human fetus brain cDNA library was used as the template to carry out PCR. The obtained PCR product was used as a probe to screen the cDNA library and SGC30343 (clone 31) was cloned.

Two RACE primers of 24mer toward the 5'-direction (5'race1(n.t.124) and 5'race2 (n.t.60)) were prepared from the sequence of SGC30343 (clone 31; 3803 bp). Primers were designed at the T7 side of the cloning site in pBluescript which was a vector for the neuroblastoma cDNA library. The race primers and the T7 primers were used to carry out nested PCR. Specifically, in the first amplification the template DNA (neuroblastoma cDNA library), the primers (vector-T7, 5'racel), dNTP, TaqDNA polymerase and others were used to carry out PCR according to a conventional method. In the second amplification, the template DNA (the first amplified product diluted to 1/100 for use), the primers (vector-T7, 5'race2), dNTP, TaqDNA polymerase and others were used to carry out PCR according to the conventional method in a similar manner.

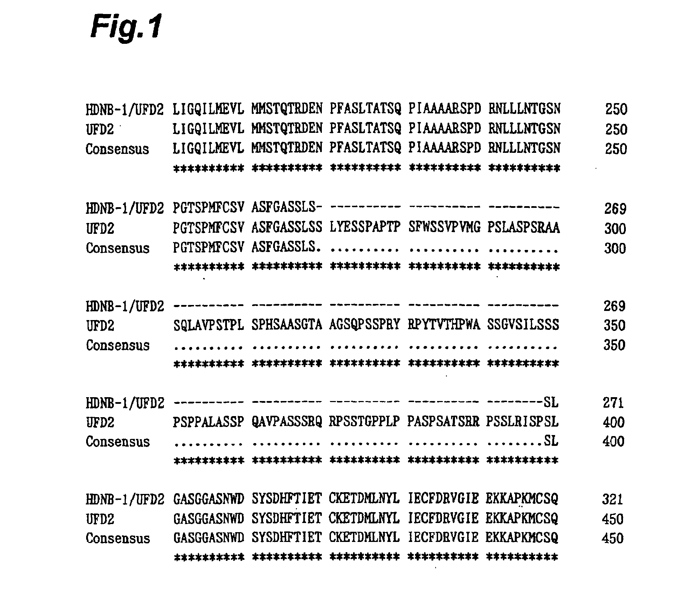

Out of the five obtained PCR products, one contained the putative 5'-upstream (of the race primer) region of SGC30343. Thus, an 840 bp (5'-upstream) was isolated. An in-frame stop codon (135 bp upstream Met) was found in the amino acid sequence. Consequently, SGC30343 was found to be 4636 bp (1173 amino acids). This PCR product was digested with a restriction enzyme to produce a fragment containing a start codon, and it was ligated to clone 31, from which the full-length HDNB-1/UFD2 was obtained. The base sequence HDNB-1/UFD2, including 5'-non-tranlational region is set forth in SEQ ID NO:1; the amino acid sequence of the translated protein is set forth in SEQ ID NO: 2, respectively. The base sequence set forth in SEQ ID NO:1 was registered with DDBJ, GenBank and EMBL (Accession No. AB028839). The base sequence set forth in SEQ ID NO:1 is shorter than the published sequence of human UFD2 (AF043117, loc. cit.) by 387 bp. In human UFD2 129 amino acids have been inserted in-frame after the 270th codon of HDNB-1/UFD2. Fig. 1 shows a comparison by alignment of the amino acid sequences between the two in the region. The base sequence set forth in SEQ ID NO:1 shows homology of 97.4% with the base sequence registered for murine UFD2.

### (Example 2) Tissue-dependent expression of HDNB-1/UFD2 gene

Tissue-dependent expression of HDNB-1/UFD2 gene in normal human tissue mRNA (Clontech Laboratories. Inc.) was studied according to the semi-quantitative RT-PCR method.

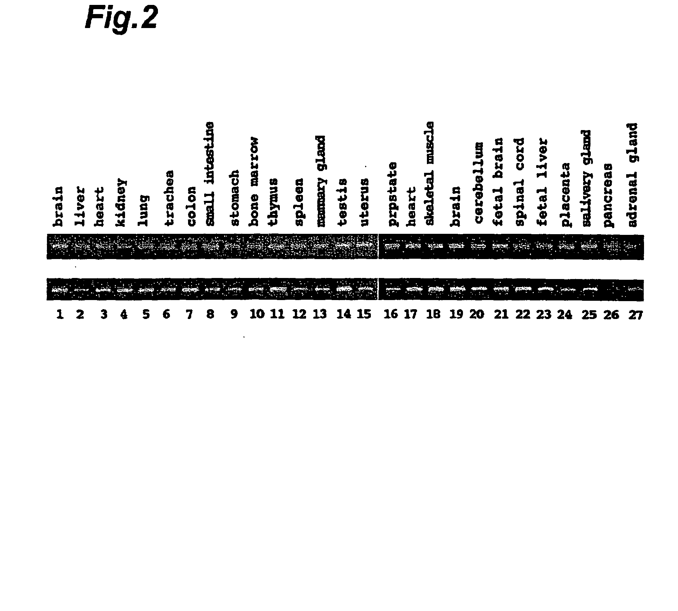

First, a portion of the HDNB-1/UFD2 gene obtained in Example 1 was used to prepare appropriate PCR primers. Separately, the mRNA was reverse-transcribed to cDNA according to a conventional method. PCR was carried out with rTaq (Takara Shuzo Co. Ltd.) under the conditions described in the following: specifically, 2 µl cDNA, 5 µl sterilized distilled water, 1 µl 10xrTaq buffer, 1 µl of 2mM dNTPs, each 0.5 µl synthesized primer set, and 0.5 µl rTaq were mixed. After this mixed solution was denatured at 95 °c for 2 minutes, 35 cycles were repeated, one cycle of which consisted of 95 °c (15 seconds), 58 °c (15 seconds), and 72 °c (20 seconds), and the solution was allowed to stand at 72 °c for 20 minutes. GAPDH was used as a positive control and similar PCR was carried out. These reaction solutions were electrophoresed on a 2.5% agarose gel. Results are shown in Fig. 2. In the figure the upper part represents the detection results of HDNB-1/UFD2 gene, while the lower part represents the detection results of control GAPDH. The expression of HDNB-1/UFD2 gene displays no particular tissue-dependence and appeared in almost all human organs.

### (Example 3) Ubiquitin ligase activity of HDNB-1/UFD2

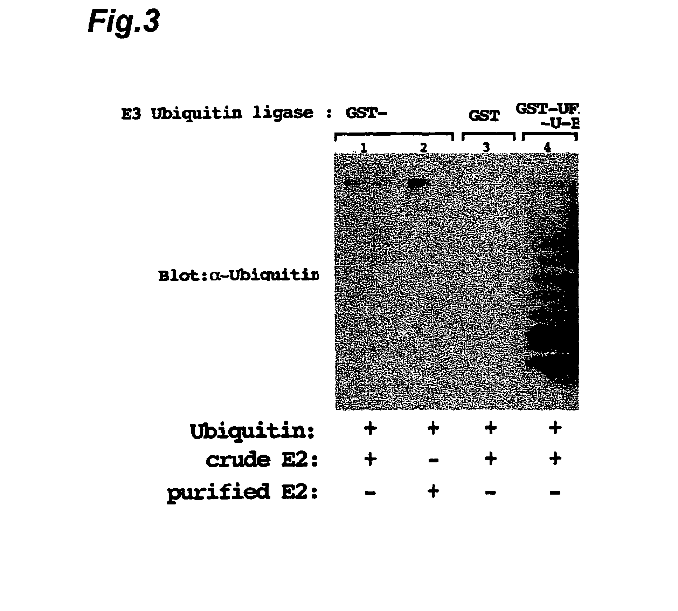

The ubiquitin ligase activity of HDNB-1/UFD2 was tested in *in vitro* ubiquitin addition reconstruction system. Specifically, ATP, a cell extract of E. *coli* (substitute substrate, crude E2), E. *coli*-derived E2 (UbcH7 purified form), E1 extracted from yeast, ubiquitin (purified protein), and E3 (ubiquitin ligase) were incubated in a tube at 37 °c for 2 hours. The reaction system contained in addition to ubiquitin enzyme, 25 mM Tris (pH 7.5), 50 mM NaCl, 10 mM MgCl₂, 10 mM DTT, and 10 mM ATP. The reaction was separated on SDS-PAGE under reductive conditions and blotted with anti-ubiquitin antibodies. When E3 activity was noted, a smear band would be detected from the low molecular weight region to the high molecular weight region. In E3, NEDD4 and HDNB-1/UFD2, which were known to have comparatively strong ubiquitin ligase activity, were used as positive controls in the forms of GST-fused proteins. The U-box region locating at the C-terminus of HDNB-1/UFD2 was formed into the GST-fused protein in the latter. Results are shown in Fig. 3. In the figure, Lane 1 represents the results obtained with the use of NEDD4, where the smear-like band was detected. Lane 2 also represents the results obtained with the use of NEDD4, where incubation was carried out exclusive of the E. *coli* extract as the substitute substrate. A smear-like band was detected. Lane 3 represents the results obtained with the use of GST protein only (negative control), where no smear-like band was detected. Lane 4 represents the results obtained with HDNB-1/UFD2, where smear-like bands were detected across the entire lane. These results have revealed strong ubiquitin ligase activity of HDNB-1/UFD2. E4 is known to show the same activity as in the ubiquitin synthesis system in the absence of E3 (Fig. 3 in Koegl et al., loc. cit.), which is consistent with the results described above.

### (Example 4) Insertion of PEX14 gene or HDNB-1/UFD2 gene into neuroblastoma cell line C201

### (Recombinant viral vector)

The full-length ORFs' of PEX14 (obtained from professor Yukio Fujiki at Department of Biology, Faculty of Science, Kyushu University and sequenced) and HDNB-1/UFD2 (Example 1) were made blunt-ended with Klenow fragment, and then were respectively incorporated into the HpaI site of retroviral vector pLXN using a Takara Ligation kit ver2. Similarly, PEX14 or HDNB-1/UDF2 was transformed into E. *coli* DH5α and plasmid was extracted from the colonies that had expressed the ampicillin resistant gene. After the direction of the inserted gene was confirmed by restriction enzymes, the plasmid was subjected to the experiments described below.

### (Transfection)

The respective viral vectors were transfected into ecotrophic packaging cells Ψ2, which had been cultured to be subconfluent in a 6-cm dish using GibcoBRL fungene 6.

### (Infection)

Amphotrophic packaging cells (PA317) were infected with the Ψ2 viral cells that had been selected with G418 (400 µg/ml) for 2 weeks and high titer viral solutions were produced. C201 cell line was infected with the viruses and a plural number of neomycin resistant clones were obtained after selection with G418. The obtained clones were designated C201(PEX14) and C201(HDNB-1/UFD2), respectively.

### (Northern blotting)

Total RNA was extracted from the clones obtained above (C201(PEX14) and C201(HDNB-1/UFD2)), and 30 µg was electrophoresed on an agarose gel containing 1% formalin. Subsequently, it was transferred to a nylon membrane. cDNAs of PEX14 and HDNB-1/UFD2 were labeled with ³²P to prepare probes, and these were hybridized to the membrane at 65 °c. The membrane was washed with SSC/Sarcosine at 50 °c. After drying, the membrane was exposed to a film at -70 °c, when the expression of the objective genes (PEX14 and HDNB-1/UFD2) was confirmed.

### (Example 5) X-ray radiation test and MTT assay

The cultured solutions of C201(PEX14) and C201(HDNB-1/UFD2) as obtained in Example 4 were irradiated with 60 Gy γ -ray. The irradiation conditions were as follows: with Mebatron KDZII, an output of 10 MV, Dmax25 mm and irradiation field of 40x40 cm. As a control, the culture solutions from other neuroblastoma cell lines (GOTO, CHP134, and SYSY) and cell line C201(neo) obtained from the virus cells infected only with the retroviral vector were prepared and irradiated under the same conditions as those described above.

After irradiation, the numbers of live cells were determined in each of the test culture and the control culture using the MTT method which utilized coloration of water-soluble formalin. Specifically, adherent cells in logarithmic growth phase were dispersed such that the count had been adjusted to 5x10⁴/ml. Cell dispersion was seeded into each well of a 96-well microtiter plate at 100 µl. The plate was incubated in a carbon dioxide gas incubator overnight. Cell counting kit-8 (Dojindo Laboratories) solution, 10 µl, was added to each well and incubation was carried out in the incubator for 2 hours. A microplate reader was used to measure absorbance at 450 nm (reference wavelength: 595 nm). The measurement was made three times and average values and standard deviations were plotted in graphs.

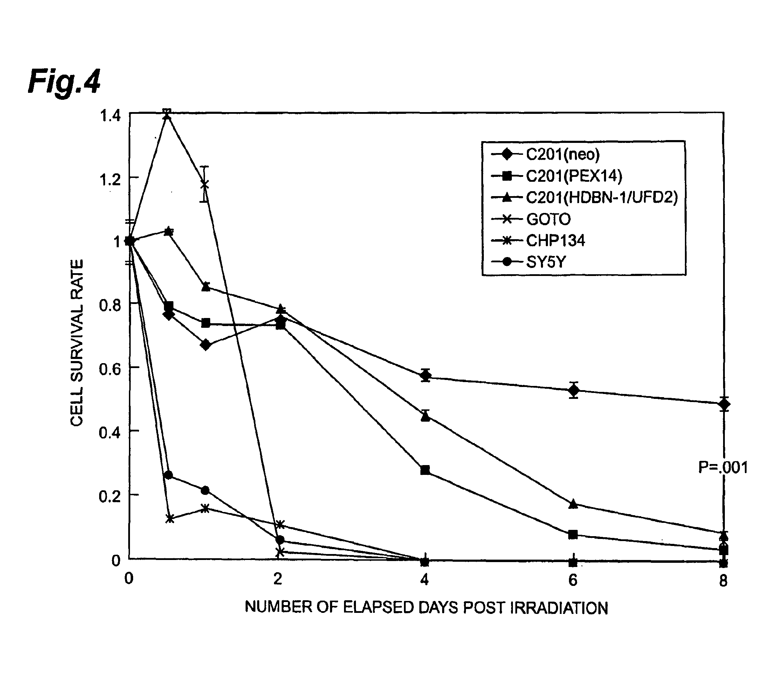

Fig. 4 is a plot of the measurement results as cell survival rate against the number of elapsed days post-irradiation. As is clearly shown in Fig. 4, almost all cells dyed within two days after irradiation in the tested neuroblastoma cell lines except C201. By contrast, about 60% cells survived even after one week post-irradiation in cell line C201(neo).

However, about 80% cells dyed on the 6th day post-irradiation in C201(PEX14) and C201(HDNB-1/UFD2) cells, where PEX14 or HDNB-1/UFD2 genes were inserted into C201 cells through transfection. It was thus found that the radiosensitivity of the gene-transferred C201 cells was significantly increased as compared to C201(neo) as the control.

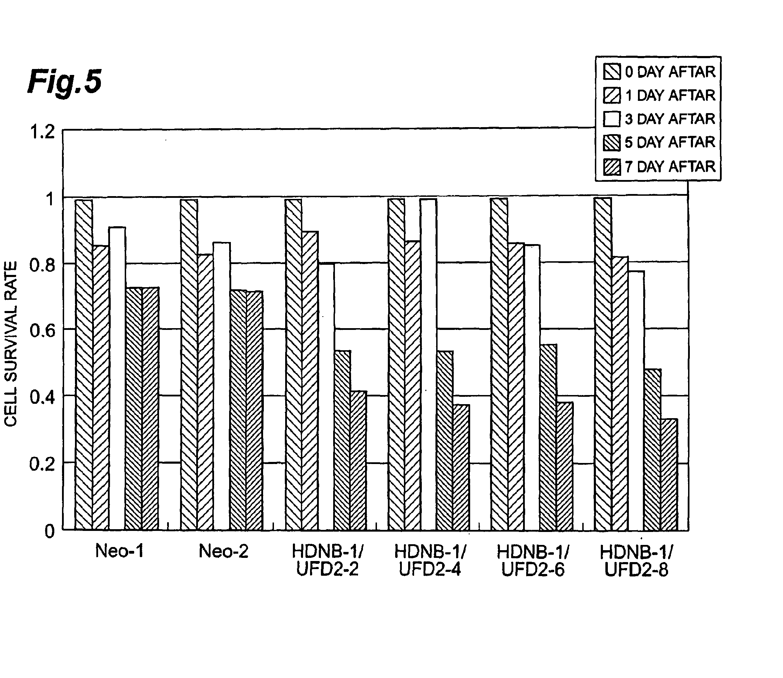

To further validate the significant difference in radiosensitivity, the radiosensitivity was compared and examined between plural C201(HDNB-1/UFD2) clones and the control C201(neo)(also in a plural number). Specifically, the number of live cells was determined in each of the clones after irradiation similarly to the above, using the MTT method. Fig. 5 shows the results of measurement. The cell survival rate for C201(HDNB-1/UFD2) was evidently decreased relative to the control. This indicates that the radiosensitivity was improved in any of the tested C201 cell lines (HDNB-1/UFD2) and was increased significantly relative to the control. In addition, the cell survival rate decreased with the almost same pattern. Therefore, it has been demonstrated that regardless of cell lines, the enhancement of radiosensitivity occurred universally in C201(HDNB-1/UFD2) as a result of insertion of HDNB-1/UFD2 gene and that this gene or its expression brought about the enhancement.

### Industrial Applicability

HDNB-1/UFD2, one of the nucleic acids of this invention, is an UFD2 associated protein expressed in neuroblastoma; therefore, the invention has elucidated the base sequence information for one of the UFD2 associated genes.

The nucleic acids of this invention or their fragments may be used as probes or primers for various types of hybridization or PCR, and it will be possible to determine the sensitivity of a human neuroblastoma strain to radiotherapy by detecting in the strain, a gene of which part is formed by the nucleic acid.

When the gene according to the invention (PEX14 or HDNB-1/UFD2) is inserted into a human neuroblastoma strain with lowered radiosensitivity, the radiosensitivity will be markedly enhanced. Thus, a patient suffering from such neuroblastoma is administered with the aforementioned gene or the protein encoded by the gene and then radiotherapy is conducted on the patient, resulting in improved therapeutic effect.

## Claims

1. A nucleic acid having the base sequence set forth in SEQ ID NO:1 in the Sequence Listing.

2. A nucleic acid encoding a protein comprising the amino acid sequence set forth in SEQ ID NO:2 in the Sequence Listing.

3. A protein having the amino acid sequence set forth in SEQ ID NO:2 in the Sequence Listing.

4. A radiosensitivity enhancer comprising as the effective ingredient, a nucleic acid having the base sequence set forth in SEQ ID NO:1 in the Sequence Listing.

5. A radiosensitivity enhancer comprising as the effective ingredient, a nucleic acid encoding a protein comprising the amino acid sequence set forth in SEQ ID NO:2 in the Sequence Listing.

6. A radiosensitivity enhancer comprising as the effective ingredient, a nucleic acid having the base sequence set forth in SEQ ID NO:3 in the Sequence Listing.

7. A radiosensitivity enhancer comprising as the effective ingredient, a protein having the amino acid sequence set forth in SEQ ID NO:2 in the Sequence Listing or a pharmaceutically acceptable salt thereof.

8. A radiosensitivity enhancer comprising as the effective ingredient, a partial peptide which is a functionally effective fragment of the protein having the amino acid sequence set forth in SEQ ID NO:2 in the Sequence Listing or a pharmaceutically acceptable salt thereof.

9. A radiosensitivity enhancer comprising as the effective ingredient, a protein having the amino acid sequence set forth in SEQ ID NO:4 in the Sequence Listing or a pharmaceutically acceptable salt thereof.

10. A radiosensitivity enhancer comprising as the effective ingredient, a partial peptide which is a functionally effective fragment of the protein having the amino acid sequence set forth in SEQ ID NO:4 in the Sequence Listing or a pharmaceutically acceptable salt thereof.

11. A recombinant viral vector comprising a nucleic acid having the base sequence set forth in SEQ ID NO:1 or SEQ ID NO:3 in the Sequence Listing alone, or in combination with an appropriate regulatory and expression control element operably linked thereto.

12. A nucleic acid probe comprising the following nucleic acid (a) or (b):
(a) Nucleic acid having a portion of the base sequence set forth in SEQ ID NO:1 in the Sequence Listing, or a base sequence complementary thereto;
(b) Nucleic acid which hybridizes to nucleic acid having the base sequence set forth in SEQ ID NO:1 in the Sequence Listing under stringent conditions, or a base sequence complementary thereto.

13. A nucleic acid probe comprising the following nucleic acid (a) or (b):
(a) Nucleic acid having a portion of the base sequence set forth in SEQ ID NO:3 in the Sequence Listing, or a base sequence complementary thereto;
(b) Nucleic acid which hybridizes to nucleic acid having the base sequence set forth in SEQ ID NO:3 in the Sequence Listing under stringent conditions, .

14. A method for determining the sensitivity of a clinically isolated human neuroblastoma strain to radiotherapy, the method comprising detecting a nucleic acid having a base sequence set forth in SEQ ID NO:1 or SEQ ID NO:3 or a fragment thereof in the human neuroblastoma strain.

15. A radiation-protecting agent comprising as the effective ingredient, an antisense nucleic acid to the nucleic acid having the base sequence set forth in SEQ ID NO:1 or SEQ ID NO:3 in the Sequence Listing.


SEQUENCE LISTING

<110> Hisamitsu Pharmaceutical Co., Inc.

<120> Radiosensitivity enhancers, Radiosensitivity-enhancing nucleic acids
and proteins in cancer radiotherapy

<130> FP01-4019-00

<150> JP 2000-351630
<151> 2000-11-17

<160> 4

<170> PatentIn Ver. 2.1

<210> 1
<211> 4636
<212> DNA
<213> Homo sapiens

<400> 1
gcacgtggtt ttgatgaata atacttggtg gggcgagggg gaaagagtag aggtggaggg 60
gtaggaggat ttactcttcc agcgagagct acgcgcatcc catcctcccc ctccccccta 120
cccgggctcc ggcgtggagg cggggcgtgg ccggcctgct ttgggagggg aggggcttcc 180
cttacagtgc tgggctctgc caggacggct gtggggtcgc cttacctcgg ggtatccact 240
ctgcagtcga ccagttcccg ccaggagcaa agggtaggaa ggagagcagg atctgctgta 300
ggaacgcagc taccgcgcca ctatcacgaa gaaacagcag gctcggggca cgagacgaac 360
tggagaccgc gctgcctagc tgggtaactt gggaagcaga gggtaataag tggcgcctta 420

agacaaccct gtagcagcag cagtggcggc caaaggaggn tgctcaggga acaagcggct 480
gtagtagtct gtggggcgac tggagtgacc gaagccaagg cagtttagtg cctctcgtgt 540
tcttattttt taacctcttg actatgcaat tctgaaacct cccccattcg ggggaccaga 600
cggcctgata gacaccttcc actctccttc ctcccgccgt ggtctcgaga acagaaggat 660
ctctccttaa cgcctttcac cattaagagg aaagcgatgg aggagctgag cgctgatgag 720
attcgacgga ggcgccttgc acgacttgct ggtggacaga cctctcagcc aaccacccca 780
ctcacctctc cccagaggga gaaccctccg gggcctccca tagcggcatc agccccagga 840
ccctctcaga gtcttggtct caatgtccac aacatgaccc cagctacctc cccaataggt 900
gcatcaggag tagcccatcg aagccagagc agtgaaggag tcagttctct cagcagctcg 960
ccctctaata gccttgaaac gcaatctcag tctctctcac gttcccagag catggatatc 1020
gatggtgtct catgtgagaa aagcatgtcc caggtggatg tggattcagg aattgaaaac 1080
atggaggttg atgaaaatga tcgaagagaa aagcggagcc tcagtgataa ggagccttcc 1140
tcgggccctg aagtgtctga agagcaggcc ttacagctgg tctgtaagat cttccgtgtc 1200
tcttggaagg accgggacag agatgtcatc tttctttctt ctctttctgc acagtttaag 1260
cagaacccaa aagaagtatt ctccgatttt aaggacttga ttggccagat tttaatggaa 1320
gtgctaatga tgtccactca gaccagagat gaaaacccat ttgccagtct gacagccaca 1380
tcacagccaa ttgctgcagc agcacggtca ccagacagaa atctcttgct aaacactggc 1440
tccaatccag gaacaagccc catgttctgc agcgtggctt cctttggtgc cagctctttg 1500
tctagtttgg gagcctctgg tggagcaagt aattgggatt cctacagtga ccatttcacc 1560
attgaaacct gcaaagagac agatatgctg aactacctca tcgagtgttt cgaccgagtt 1620
ggaatagagg aaaaaaaagc accaaagatg tgcagccagc cagcagtcag ccagcttctg 1680
agcaacatcc gctcacagtg catatcccat actgctttag tactacaagg ctccctaaca 1740
cagcccaggt ccttgcagca gccgtccttc ctagtgccgt atatgctgtg taggaatctc 1800
ccatatggct tcattcagga actggtgaga accactcacc aggatgaaga agtgttcaag 1860
cagatattta tccccatttt acaaggcctg gctcttgctg ccaaagagtg ctccctcgac 1920
agtgactact ttaaataccc cctcatggca ctaggtgagc tctgtgaaac caagtttggg 1980
aagacacacc ctgtgtgcaa tttggttgct tctttgcggt tgtggttgcc gaaatcctta 2040
agtcctggct gtgggcggga gctgcagaga ctctcttact taggggcttt ctttagcttc 2100
tcagtctttg cagaagatga tgttaaagtg gttgaaaaat acttctcagg gcctgccatt 2160

accctggaaa acactcgtgt ggttagccaa tcattgcagc attacttaga gctcggaagg 2220
caagagcttt ttaagattct gcatagtatt ttgttaaatg gcgaaacccg tgaggctgct 2280
ctcagttaca tggcggctgt cgtcaatgcc aatatgaaga aagcacagat gcagacagat 2340
gatagattgg tgtctacaga tggatttatg ctgaatttcc tttgggtact gcagcagcta 2400
agtacaaaaa tcaagttaga aacagttgat cccacgtata tttttcaccc aagatgtcgg 2460
attactcttc ccaatgatga gacgcgtgtg aatgcaacga tggaagatgt gaatgactgg 2520
ctgactgaac tctatggcga tcagcctcca ttttctgagc cgaaattccc tacggagtgc 2580
ttctttctca ccctgcatgc tcaccacctc tctattctgc ctagttgccg tcgctatatc 2640
cgcagactcc gggctatccg ggagctcaat agaactgtag aagatttgaa aaataatgaa 2700
agccaatgga aagattcccc actggcaact agacaccgcg aaatgctgaa gcgctgtaaa 2760
actcagctta agaaactggt acggtgcaag gcctgtgctg atgctggcct acttgacgag 2820
agcttcctga gaagatgtct gaatttttat ggccttctca ttcagctgct gctccgcatc 2880
ctggaccccg catatcccga tataacactg cctttaaatt cagatgtccc caaggtattt 2940
gcagcgttgc ctgagtttta tgtagaagat gttgcagaat tttatttttt tattgtacaa 3000
tactctcccc aggcgcttta tgagccctgt actcaggata ttgtgatgtt ccttgttgtg 3060
atgttgtgca accagaacta catccgaaac ccatatttgg tggccaaact ggtagaagtc 3120
atgtttatga ccaaccctgc tgttcagcca cgaacccaga agtttttgga aatgattgag 3180
aaccatcctc tctccaccaa gttgttggta ccttccctga tgaagtttta tacagatgtt 3240
gagcataccg gagccaccag tgagttttat gacaagttca caattcgcta tcatattagc 3300
accattttta aaagcctttg gcaaaacata gctcaccatg gcacctttat ggaggagttc 3360
aactccggga agcagtttgt tcgctatata aacatgttga taaacgacac gacgtttttg 3420
ctcgatgaaa gtctggagtc tctgaagcga atccatgaag tgcaggaaga gatgaagaac 3480
aaagaacagt gggaccagtt gccccgggat cagcagcagg ctcgtcagtc tcagcttgct 3540
caggatgagc gtgtgtcccg ctcttacctc gccctggcca ccgaaaccgt ggacatgttc 3600
cacatcctca cgaagcaggt ccagaagccc ttcctcagac cggagcttgg accccgattg 3660
gctgcaatgc tgaactttaa tcttcagcaa ctttgtggcc ccaagtgccg tgacctgaaa 3720
gttgaaaacc ctgagaaata cggctttgaa ccaaagaagc tgttggacca actgacggat 3780
atttacttac agctggactg tgctcggttc gcgaaagcca ttgctgacga ccagagatcc 3840
tacagtaagg aattgtttga agaagttatt tcaaagatgc ggaaggcagg gatcaaatcc 3900

acaatagcaa tagaaaaatt taagctgctc gccgagaaag tggaggagat agtggccaag 3960

aacgcacgcg cagaaatcga ctacagcgac gctcctgatg agttcagaga ccctctgatg 4020

gacaccctca tgacagaccc cgtgcggctg ccctctggca ccatcatgga ccgctccatc 4080

atcctgcggc acctgctcaa ctcccccacg gaccccttca accggcagac gctgacagag 4140

agcatgctgg aaccagtgcc agaactgaaa gagcagattc aggcgtggat gagagagaaa 4200

cagaacagcg atcactaaac cgttccgccg cccaccctct gctagacaca gccaaggcca 4260

acgaggcaag cagaagcagc ggccgcagcg aagctgccgt tcatgtgttg gaggccaaat 4320

gtggcaaacc aaccccaggc ccacccagag cgagcaaacg ctgagacctg aaaggacatg 4380

gatgagaaga ggagcccgct tcctgtacat atatttaagt gacaaacacg gtcaaaagct 4440

taagggacag gttttatggt tgcttgtgta ataaagcatg tccttcgtat gtcacagttt 4500

ggggcaacgg aagtctttta gtgatggcta atgggtctgg gcagcatccc ttcatgaatt 4560

tttttttaat ccaatatccg ttgatttgat tgtgattaga gaaccttgga cattttgctg 4620

ctaaagaatg gaattc 4636

<210> 2

<211> 1173

<212> PRT

<213> Homo sapiens

<400> 2

Met Glu Glu Leu Ser Ala Asp Glu Ile Arg Arg Arg Arg Leu Ala Arg

1               5                   10                  15

Leu Ala Gly Gly Gln Thr Ser Gln Pro Thr Thr Pro Leu Thr Ser Pro

            20                  25                  30

Gln Arg Glu Asn Pro Pro Gly Pro Pro Ile Ala Ala Ser Ala Pro Gly

        35                  40                  45

Pro Ser Gln Ser Leu Gly Leu Asn Val His Asn Met Thr Pro Ala Thr
    50                  55                  60

Ser Pro Ile Gly Ala Ser Gly Val Ala His Arg Ser Gln Ser Ser Glu
65                  70                  75                  80

Gly Val Ser Ser Leu Ser Ser Ser Pro Ser Asn Ser Leu Glu Thr Gln
                85                  90                  95

Ser Gln Ser Leu Ser Arg Ser Gln Ser Met Asp Ile Asp Gly Val Ser
            100                 105                 110

Cys Glu Lys Ser Met Ser Gln Val Asp Val Asp Ser Gly Ile Glu Asn
        115                 120                 125

Met Glu Val Asp Glu Asn Asp Arg Arg Glu Lys Arg Ser Leu Ser Asp
    130                 135                 140

Lys Glu Pro Ser Ser Gly Pro Glu Val Ser Glu Glu Gln Ala Leu Gln
145                 150                 155                 160

Leu Val Cys Lys Ile Phe Arg Val Ser Trp Lys Asp Arg Asp Arg Asp
                165                 170                 175

Val Ile Phe Leu Ser Ser Leu Ser Ala Gln Phe Lys Gln Asn Pro Lys
            180                 185                 190

Glu Val Phe Ser Asp Phe Lys Asp Leu Ile Gly Gln Ile Leu Met Glu

        195                 200                 205

Val Leu Met Met Ser Thr Gln Thr Arg Asp Glu Asn Pro Phe Ala Ser
    210                 215                 220

Leu Thr Ala Thr Ser Gln Pro Ile Ala Ala Ala Ala Arg Ser Pro Asp
225                 230                 235                 240

Arg Asn Leu Leu Leu Asn Thr Gly Ser Asn Pro Gly Thr Ser Pro Met
                245                 250                 255

Phe Cys Ser Val Ala Ser Phe Gly Ala Ser Ser Leu Ser Ser Leu Gly
            260                 265                 270

Ala Ser Gly Gly Ala Ser Asn Trp Asp Ser Tyr Ser Asp His Phe Thr
        275                 280                 285

Ile Glu Thr Cys Lys Glu Thr Asp Met Leu Asn Tyr Leu Ile Glu Cys
    290                 295                 300

Phe Asp Arg Val Gly Ile Glu Glu Lys Lys Ala Pro Lys Met Cys Ser
305                 310                 315                 320

Gln Pro Ala Val Ser Gln Leu Leu Ser Asn Ile Arg Ser Gln Cys Ile
                325                 330                 335

Ser His Thr Ala Leu Val Leu Gln Gly Ser Leu Thr Gln Pro Arg Ser
            340                 345                 350

Leu Gln Gln Pro Ser Phe Leu Val Pro Tyr Met Leu Cys Arg Asn Leu
        355                 360                 365
Pro Tyr Gly Phe Ile Gln Glu Leu Val Arg Thr Thr His Gln Asp Glu
    370                 375                 380
Glu Val Phe Lys Gln Ile Phe Ile Pro Ile Leu Gln Gly Leu Ala Leu
385                 390                 395                 400
Ala Ala Lys Glu Cys Ser Leu Asp Ser Asp Tyr Phe Lys Tyr Pro Leu
                405                 410                 415
Met Ala Leu Gly Glu Leu Cys Glu Thr Lys Phe Gly Lys Thr His Pro
            420                 425                 430
Val Cys Asn Leu Val Ala Ser Leu Arg Leu Trp Leu Pro Lys Ser Leu
        435                 440                 445
Ser Pro Gly Cys Gly Arg Glu Leu Gln Arg Leu Ser Tyr Leu Gly Ala
    450                 455                 460
Phe Phe Ser Phe Ser Val Phe Ala Glu Asp Asp Val Lys Val Val Glu
465                 470                 475                 480
Lys Tyr Phe Ser Gly Pro Ala Ile Thr Leu Glu Asn Thr Arg Val Val
                485                 490                 495
Ser Gln Ser Leu Gln His Tyr Leu Glu Leu Gly Arg Gln Glu Leu Phe
            500                 505                 510

Lys Ile Leu His Ser Ile Leu Leu Asn Gly Glu Thr Arg Glu Ala Ala
        515                 520                 525
Leu Ser Tyr Met Ala Ala Val Val Asn Ala Asn Met Lys Lys Ala Gln
    530                 535                 540
Met Gln Thr Asp Asp Arg Leu Val Ser Thr Asp Gly Phe Met Leu Asn
545                 550                 555                 560
Phe Leu Trp Val Leu Gln Gln Leu Ser Thr Lys Ile Lys Leu Glu Thr
                565                 570                 575
Val Asp Pro Thr Tyr Ile Phe His Pro Arg Cys Arg Ile Thr Leu Pro
            580                 585                 590
Asn Asp Glu Thr Arg Val Asn Ala Thr Met Glu Asp Val Asn Asp Trp
        595                 600                 605
Leu Thr Glu Leu Tyr Gly Asp Gln Pro Pro Phe Ser Glu Pro Lys Phe
    610                 615                 620
Pro Thr Glu Cys Phe Phe Leu Thr Leu His Ala His His Leu Ser Ile
625                 630                 635                 640
Leu Pro Ser Cys Arg Arg Tyr Ile Arg Arg Leu Arg Ala Ile Arg Glu
                645                 650                 655
Leu Asn Arg Thr Val Glu Asp Leu Lys Asn Asn Glu Ser Gln Trp Lys

            660                 665                 670

Asp Ser Pro Leu Ala Thr Arg His Arg Glu Met Leu Lys Arg Cys Lys

        675                 680                 685

Thr Gln Leu Lys Lys Leu Val Arg Cys Lys Ala Cys Ala Asp Ala Gly

    690                 695                 700

Leu Leu Asp Glu Ser Phe Leu Arg Arg Cys Leu Asn Phe Tyr Gly Leu

705                 710                 715                 720

Leu Ile Gln Leu Leu Leu Arg Ile Leu Asp Pro Ala Tyr Pro Asp Ile

                725                 730                 735

Thr Leu Pro Leu Asn Ser Asp Val Pro Lys Val Phe Ala Ala Leu Pro

            740                 745                 750

Glu Phe Tyr Val Glu Asp Val Ala Glu Phe Leu Phe Phe Ile Val Gln

        755                 760                 765

Tyr Ser Pro Gln Ala Leu Tyr Glu Pro Cys Thr Gln Asp Ile Val Met

    770                 775                 780

Phe Leu Val Val Met Leu Cys Asn Gln Asn Tyr Ile Arg Asn Pro Tyr

785                 790                 795                 800

Leu Val Ala Lys Leu Val Glu Val Met Phe Met Thr Asn Pro Ala Val

                805                 810                 815

Gln Pro Arg Thr Gln Lys Phe Phe Glu Met Ile Glu Asn His Pro Leu
            820                 825                 830

Ser Thr Lys Leu Leu Val Pro Ser Leu Met Lys Phe Tyr Thr Asp Val
        835                 840                 845

Glu His Thr Gly Ala Thr Ser Glu Phe Tyr Asp Lys Phe Thr Ile Arg
    850                 855                 860

Tyr His Ile Ser Thr Ile Phe Lys Ser Leu Trp Gln Asn Ile Ala His
865                 870                 875                 880

His Gly Thr Phe Met Glu Glu Phe Asn Ser Gly Lys Gln Phe Val Arg
                885                 890                 895

Tyr Ile Asn Met Leu Ile Asn Asp Thr Thr Phe Leu Leu Asp Glu Ser
            900                 905                 910

Leu Glu Ser Leu Lys Arg Ile His Glu Val Gln Glu Glu Met Lys Asn
        915                 920                 925

Lys Glu Gln Trp Asp Gln Leu Pro Arg Asp Gln Gln Gln Ala Arg Gln
    930                 935                 940

Ser Gln Leu Ala Gln Asp Glu Arg Val Ser Arg Ser Tyr Leu Ala Leu
945                 950                 955                 960

Ala Thr Glu Thr Val Asp Met Phe His Ile Leu Thr Lys Gln Val Gln
                965                 970                 975

Lys Pro Phe Leu Arg Pro Glu Leu Gly Pro Arg Leu Ala Ala Met Leu
            980                 985                 990

Asn Phe Asn Leu Gln Gln Leu Cys Gly Pro Lys Cys Arg Asp Leu Lys
        995                 1000                1005

Val Glu Asn Pro Glu Lys Tyr Gly Phe Glu Pro Lys Lys Leu Leu Asp
    1010                1015                1020

Gln Leu Thr Asp Ile Tyr Leu Gln Leu Asp Cys Ala Arg Phe Ala Lys
1025                1030                1035                1040

Ala Ile Ala Asp Asp Gln Arg Ser Tyr Ser Lys Glu Leu Phe Glu Glu
                1045                1050                1055

Val Ile Ser Lys Met Arg Lys Ala Gly Ile Lys Ser Thr Ile Ala Ile
            1060                1065                1070

Glu Lys Phe Lys Leu Leu Ala Glu Lys Val Glu Glu Ile Val Ala Lys
        1075                1080                1085

Asn Ala Arg Ala Glu Ile Asp Tyr Ser Asp Ala Pro Asp Glu Phe Arg
    1090                1095                1100

Asp Pro Leu Met Asp Thr Leu Met Thr Asp Pro Val Arg Leu Pro Ser
1105                1110                1115                1120

Gly Thr Ile Met Asp Arg Ser Ile Ile Leu Arg His Leu Leu Asn Ser

1125                1130                1135

Pro Thr Asp Pro Phe Asn Arg Gln Thr Leu Thr Glu Ser Met Leu Glu

        1140                1145                1150

Pro Val Pro Glu Leu Lys Glu Gln Ile Gln Ala Trp Met Arg Glu Lys

    1155                1160                1165

Gln Asn Ser Asp His

    1170

<210> 3

<211> 1908

<212> DNA

<213> Homo sapiens

<400> 3

gaaagatggc gtcctcggag caggcagagc agccgagcca gccaagctct actccaggaa 60

gtgaaaatgt gctgcctcga gagccgctga ttgccacggc agtgaagttt ctacagaatt 120

cccgggtccg ccagagccca cttgcaacca ggagagcatt tctaaagaag aaagggctga 180

cagatgaaga gattgatatg gccttccagc agtcgggcac tgctgccgat gagccttcgt 240

ccttgggccc agccacacag gtggttcctg tccagccccc tcacctcata tctcagccat 300

acagtcccgc aggctcccga tggcgagatt acggcgccct ggccatcatc atggcaggca 360

ttgcatttgg ctttcaccag ctctacaaga aatacctgct ccccctcatc ctgggcggcc 420

gagaggacag aaagcagctg gagaggatgg aggccggtct ctctgagctg agtggcagcg 480

tggcccagac agtgactcag ttacagacga ccctcgcctc cgtccaggag ctgctgattc 540

agcagcagca gaagatccag gagcttgccc acgagctggc cgctgccaag gccaccacat 600

ccaccaactg gatcctggag tcccagaata tcaacgaact caagtccgaa attaactcct 660

tgaaagggct tcttttaaat cggaggcagt tccctccatc cccatcagcc ccgaagatcc 720
cctcctggca gatcccagtc aagtcaccgt caccctccag ccctgcggcc gtgaaccacc 780
acagcagcag cgacatctca cctgtcagca acgagtccac gtcgtcctcg cctgggaagg 840
agggccacag ccccgagggc tccacggtca cctaccactt gctgggcccc caggaggaag 900
gcgagggggt ggtggacgtc aagggccagg tgcggatgga ggtgcaaggc gaggaggaga 960
agagggagga caaggaggac gaggaggatg aggaggatga tgatgtgagc catgtggacg 1020
aggaggactg cctgggggtg cagagggagg accgccgggg cggggatggg cagatcaacg 1080
agcaggtgga gaagctgcgg cggcccgagg gcgccagcaa cgagagtgag cgggactagg 1140
gctgcgcctg ctgcctccag ccctgaggat ggcatctagt gtgcccgtgc gtggccatac 1200
cctgcctccc tctctggccc tgggagggca gcttggagcc caggtagggg gcagagctgt 1260
cctcagctgc actgcggcct ggtggcagtg tggggagtca cacttctgtc cacctggcct 1320
cctctcgcct ggccgccagc cccagcccca gccccagccc caggcccagc tgcctttggc 1380
tttgatctca agtcaggctg aaggcagcga agcctcgggg cccaagcccc tccccagccc 1440
cctctcccgg acagacgcct tgcccagggt gtgtttgctg agtgtcttga ctaccgtgac 1500
accacgcatg gccagagcta gcgtccctac tgcctcccga ctcctcagtg gaggaggagc 1560
tgcggtccct ctggtgtctg ccatccccct ccctccctgg gcccggccct ggacccgtca 1620
ggtgcctgtc cccagcccca accccactca tgccccgtcg tcctcccaga caaatgaaac 1680
cacgctgcgc ttccgatgcc cccgcttgcc gtgtaatggt tcagctaatc ccatggcgag 1740
atgggggctc attccggagg aggagccagg cagcagggcc ttcctgacca acagccagtt 1800
ttgtccttcc ccccaggaaa aaaatgttca tttgtgtgat catgtataga cctcagaacg 1860
gaagatagga ctgtatataa ttgtaataaa taccagttgc cactattt 1908

<210> 4
<211> 377
<212> PRT
<213> Homo sapiens

<400> 4

Met Ala Ser Ser Glu Gln Ala Glu Gln Pro Ser Gln Pro Ser Ser Thr
1               5                   10                  15

Pro Gly Ser Glu Asn Val Leu Pro Arg Glu Pro Leu Ile Ala Thr Ala
            20                  25                  30

Val Lys Phe Leu Gln Asn Ser Arg Val Arg Gln Ser Pro Leu Ala Thr
        35                  40                  45

Arg Arg Ala Phe Leu Lys Lys Lys Gly Leu Thr Asp Glu Glu Ile Asp
    50                  55                  60

Met Ala Phe Gln Gln Ser Gly Thr Ala Ala Asp Glu Pro Ser Ser Leu
65                  70                  75                  80

Gly Pro Ala Thr Gln Val Val Pro Val Gln Pro Pro His Leu Ile Ser
                85                  90                  95

Gln Pro Tyr Ser Pro Ala Gly Ser Arg Trp Arg Asp Tyr Gly Ala Leu
            100                 105                 110

Ala Ile Ile Met Ala Gly Ile Ala Phe Gly Phe His Gln Leu Tyr Lys
        115                 120                 125

Lys Tyr Leu Leu Pro Leu Ile Leu Gly Gly Arg Glu Asp Arg Lys Gln
    130                 135                 140

Leu Glu Arg Met Glu Ala Gly Leu Ser Glu Leu Ser Gly Ser Val Ala
145                 150                 155                 160

Gln Thr Val Thr Gln Leu Gln Thr Thr Leu Ala Ser Val Gln Glu Leu
                165                 170                 175
Leu Ile Gln Gln Gln Gln Lys Ile Gln Glu Leu Ala His Glu Leu Ala
            180                 185                 190
Ala Ala Lys Ala Thr Thr Ser Thr Asn Trp Ile Leu Glu Ser Gln Asn
        195                 200                 205
Ile Asn Glu Leu Lys Ser Glu Ile Asn Ser Leu Lys Gly Leu Leu Leu
    210                 215                 220
Asn Arg Arg Gln Phe Pro Pro Ser Pro Ser Ala Pro Lys Ile Pro Ser
225                 230                 235                 240
Trp Gln Ile Pro Val Lys Ser Pro Ser Pro Ser Ser Pro Ala Ala Val
                245                 250                 255
Asn His His Ser Ser Ser Asp Ile Ser Pro Val Ser Asn Glu Ser Thr
            260                 265                 270
Ser Ser Ser Pro Gly Lys Glu Gly His Ser Pro Glu Gly Ser Thr Val
        275                 280                 285
Thr Tyr His Leu Leu Gly Pro Gln Glu Glu Gly Glu Gly Val Val Asp
    290                 295                 300
Val Lys Gly Gln Val Arg Met Glu Val Gln Gly Glu Glu Glu Lys Arg

305                    310                    315                    320

Glu Asp Lys Glu Asp Glu Glu Asp Glu Glu Asp Asp Asp Val Ser His

                325                    330                    335

Val Asp Glu Glu Asp Cys Leu Gly Val Gln Arg Glu Asp Arg Arg Gly

            340                    345                    350

Gly Asp Gly Gln Ile Asn Glu Gln Val Glu Lys Leu Arg Arg Pro Glu

        355                    360                    365

Gly Ala Ser Asn Glu Ser Glu Arg Asp

    370                    375